# EUROPEAN PATENT APPLICATION

(11) **EP 2 743 884 A1**
(43) Date of publication of application: **18.06.2014**
(21) Application number: 13194646.9
(22) Date of filing: 27.11.2013
(51) Int. Cl.: G06Q 50/22

(54) **System and method for improving the medical environment**

(30) Priority: 13.12.2012 US 201213713246
(71) Applicant: Elekta AB (publ), 103 93 Stockholm (SE)
(72) Inventor: Bourne, Duncan, Neil, Redhill, Surrey RH1 3BA (GB); Thompson, Daniel, Peter, Crawley, West Sussex RH10 2RR (GB)
(74) Representative: Downing, Michael Philip

(57) **Abstract**

A medical environment can be personalised by displaying themes or visual sequences during the medical procedure. For a repeating medical procedure, the process can be improved by ensuring that the same theme continues from where it left off when the patient was last present. The theme can also include elements that indicate the patient's rate or degree of progress through the medical procedure.

## Description

### FIELD OF THE INVENTION

The present invention seeks to improve the environment in which medical procedures are conducted.

### BACKGROUND ART

A person coming to a hospital for medical treatment or examination often does not feel comfortable and at ease. This is caused by a range of factors including the anxiety that may result from the medical problem that is to be treated, and by the fact that the person is often unfamiliar with the professional medical environment and/or the treatment or examination that has to be carried out. This may especially be the case with small children or older people. These negative emotions of the patient may hinder the correct conduct of the treatment or examination, thus making it necessary for the medical staff to spend more time calming and reassuring each individual patient before and during the examination or treatment. Alleviating this unease on the part of the patient would help both the patient and also the efficiency of the medical workflow.

US 7,773,777 (Raijmakers et al) describes a system and method for providing a personalized experience to a person in a medical environment. Through changes in lighting and audio solutions, together with projections of images and/or animations that are initiated by the entrance of a person (professional or patient) in a medical environment such as a radiology department, cardiology department, intensive care unit, etc., a certain ambience/atmosphere is created in this specific architectural context. This ambience can be a choice from certain predefined themes (e.g. animal drawings for children or nature images) or truly personal when personal content (e.g. images of family or vacation) is inserted in the system. The patient is able to select certain data from a collection of data, based on his or her own personal preferences, via a selection means. These data may comprise for example a composition of images and sound related to a specific theme, such as 'nature'. Based on these selected data, the control means in the system control the display of the selected data in the medical environment. In the case of for example the 'nature' theme, this means that a scene from nature is displayed with accompanying sounds, such as the ocean and the sound of waves. Throughout the examination, this scene and the sound will be provided to have a calming and relaxing effect on the patient. In this manner the patient will feel more comfortable during the examination.

US2010/0217111 discloses a manner of designing a room in which a medical imaging system for paediatric patients is located, consisting of installing a plurality of elements in or on the room and/or the medical imaging system, which collectively create a first theme that forms a physical environment at least partially surrounding a patient. This theme is presented in a manner to reduce the level of patient anxiety and fear experienced by the patient being imaged. In its widest form, the disclosed concept appears to boil down to decorating the relevant room of the clinic in order to make it less intimidating for paediatric patients, a concept which has been applied in other medical contexts for many decades.

Others focus on providing favourable lighting conditions and attractive interior design in the treatment room, asserting that these influence the well-being of patients and the daily work efficiency of the clinic. An example can be found at http://healthcare.siemens.com/accessories-oem-equipment/lighting-solutions.

### SUMMARY OF THE INVENTION

US7,773,777 is apt for one-off medical procedures such as a single scan, but does not deal well with repeating medical procedures such as repeated scans, fractionated radiotherapy treatments, or the like. In such contexts, the patient needs to return to the medical environment repeatedly, and will soon exhaust the supply of themes and/or lose interest in them.

The present invention therefore provides, in a first aspect, a method of adjusting an environment in the context of a repeating medical procedure, comprising providing (i) a treatment room in which the medical procedure can take place, the room comprising a display means, (ii) a data store, associated with the display means, and containing a plurality of displayable themes, and (iii) a patient identification means and employing these in order to identify a patient via the patient identification means, check whether records of a previous visit by that patient exist and, if no such records exist, allowing the patient to select one theme from the plurality of themes, admitting the patient into the treatment room, commencing display of the one theme via the display means, commencing a medical procedure, subsequently ceasing the medical procedure and the display of the one theme, and recording an identifier of the patient together with n identification of the one theme, or, if a record of a previous visit exists, retrieving the stored identification of the one theme, re-admitting the patient into the treatment room, re-commencing display of the one theme, and re-commencing the medical procedure.

The invention also provides a system for personalisation of a medical environment, comprising a patient support, a medical apparatus able to deliver a medical procedure to a patient located on the patient support, a display means, a data store associated with the display means and containing a plurality of displayable themes, a patient identification means, and a control means programmed to identify the patient via the patient identification means, check whether records of a previous visit by that patient exist, and if no such records exist, allow the patient to select one theme from the plurality of themes, admit the patient into the treatment room, commence display of the one theme via the display means, and commence a medical procedure, subsequently cease the medical procedure and the display of the one theme, and record in the data store an identifier of the patient together with an identification of the one theme, or, if a record of a previous visit exists, retrieve the stored identification of the one theme, re-admit the patient, into the treatment room, re-commence display of the one theme, and re-commence the medical procedure.

In a second aspect, the invention provides a method of adjusting an environment in the context of a repeating medical procedure, comprising providing a treatment room in which the medical procedure can take place, the room comprising a display means, and a data store, associated with the display means, and containing at least one displayable sequence, admitting a patient into the treatment room, commencing display of the displayable sequence via the display means, and commencing a medical procedure, subsequently ceasing the medical procedure and the display of the displayable sequence, and recording an identifier of the patient and a progress point within the displayable sequence, subsequently re-admitting the patient into the treatment room, re-commencing display of the displayable sequence from the recorded progress point, and re-commencing the medical procedure.

According to the second aspect, the invention also provides a system for personalisation of a medical environment, comprising a patient support, a medical apparatus able to deliver a medical procedure to a patient located on the patient support, a display means, a data store, associated with the display means, containing a displayable sequence, a patient identification means, and a control means programmed to commence display of the displayable sequence via the display means, and commence a medical procedure, subsequently cease the medical procedure and the display of the displayable sequence, and record in the data store an identifier of the patient and a progress point within the displayable sequence, and subsequently re-commence display of the displayable sequence from the recorded progress point, and re-commence the medical procedure.

The display means can be a projector, which may project the theme or displayable sequence onto at least one of a wall and a ceiling. Alternatively, the display means can be a wall-mounted screen.

The theme or displayable sequence can include a progress indicator for the medical procedure. The progress indicator can then reflect the progress of the part of the medical procedure relevant to the current visit, and/or the progress of the medical procedure as a whole.

An audio sequence can be played via an audio output, in conjunction with display of the theme or displayable sequence. To cater for medical procedures of different lengths, the audio sequence can have a length less than that expected for the medical procedure, and can be manipulated by inserting segments thereby to extend its length so as substantially to match the length of the medical procedure.

Other aspects of the environment could be adjusted to conform to a preference stored in association with the patient record, such as the room lighting including brightness levels and lighting pattern, audio volume, room temperature, and room humidity.

The medical procedures of particular relevance for the invention include radiotherapy treatments, CT scans, MRI scans, and CT or MRI scans provided in conjunction with radiotherapy treatment.

### BRIEF DESCRIPTION OF THE DRAWINGS

An embodiment of the present invention will now be described by way of example, with reference to the accompanying figures in which;
Figure 1 shows the layout of a radiotherapy treatment room according to the invention;
Figure 2 shows the major components of a system according to the present invention;
Figure 3 shows a process flowchart for the present invention; and
Figure 4 shows the layout of an MRI-based treatment room according to the invention.

### DETAILED DESCRIPTION OF THE EMBODIMENTS

The rooms in which clinical investigations and treatments are carried out (referred to herein as "treatment rooms") are typically decorated plainly and simply, mainly to allow for easy maintenance of the necessary standards of cleanliness. Indeed, the English-language adjective "clinical" has come to mean objective, devoid of emotion, and austere. It is being increasingly recognised that this ambience is not conducive to the type of positive attitude on the part of the patient that is known to assist with their treatment and with the efficient operation of a clinic.

The Philips "Ambient Experience" product, as described in US 7,773,777, is essentially a surround sound and projection system which can be set for a variety of themes. The disclosure of US 7,773,777 is incorporated herein by reference, and the reader is specifically directed to the disclosure of that application for a fuller understanding of the present invention. The purpose of US 7,773,777 is to make a medical imaging suite seem less threatening and the patient more relaxed while being imaged. Whilst this is an improvement on a typical clinical setting, it does not make best use of the apparatus functionality for patients who may return to the same suite repeatedly. For example, patients undergoing radiotherapy are typically treated in a series of "fractions", doses that are delivered once per day (or more frequently) until the course of treatment is complete. Such patients may tire of having to choose a theme repeatedly.

A first step is therefore to store the settings for an individual patient, so that when they return to the suite, it can be already tailored to their wishes and/or needs based on their settings from their previous session. Thus, the patient will register on arrival, so that by the time they enter the suite the control system has already retrieved their settings and the room has been given the desired appearance. The initial registration on arrival can be by a receptionist or other staff member inputting their details into the control system or a system able communicate therewith, or by presentation or scanning of an identity document, or by a biometric scan such as a hand or retinal scan. This is more welcoming to the patient and provides a visual reassurance to them that the apparatus has correctly identified them.

Once the system is able to recognise individual patients in this way, retaining details as to the ambient settings required for that patient, then the system can be extended so that the ambient settings are responsive to the patient and assist their progress through the treatment. At a simple level, as the system is then detecting the same patient returning to the clinic then it can allow for continuation between sessions, i.e. nature of the ambience is picked up from where it was left off at the end of the previous session. A more complex example is a progress indicator, which will show the patient their progress through the treatment and assist with their motivation (especially during the later parts of the process). Such indicators could be integrated into the ambience, for example in a "nature" theme, amongst a variety of species moving around the room there could be one unique individual, perhaps of a different species or a different colour, moving between two markers such as posts. The patient could be told that when the treatment fraction starts, that particular creature will start off from the first post, and that when it reaches the other post the treatment fraction will have finished. In this way they are constantly able to see the progress they are making through the fraction.

A further development of this is to have two such progress-indicating species, such as a hare and a tortoise (for example). One represents the progress of the particular fraction in question, and the other represents the progress of the treatment overall. In this case, once the hare reaches the other post, the fraction finishes. The hare will then start back at the first post at the start of the next session. Meanwhile, the tortoise had reached a point midway between the two posts by the time the fraction was complete. On return of the patient for the next fraction, the tortoise continues its progress from the point it had reached at the end of the previous fraction. Once it reaches the second post, at the end of the last fraction, the whole treatment is over. In this way the patient can keep track of both their progress through the fraction, and their progress through their entire treatment.

In a refinement of this, as the system knows which the patient is being treated and therefore can be informed as to the details of their treatment, the displayed ambience can itself be timed so as to match the length of the treatment fraction. In this way, the end of the fraction can coincide with a suitable break point in the displayed ambience. That break point could form a "cliff-hanger", i.e. something that asks a question or leaves a knowledge gap that will be filled during the next fraction thereby easing the patient into the next session.

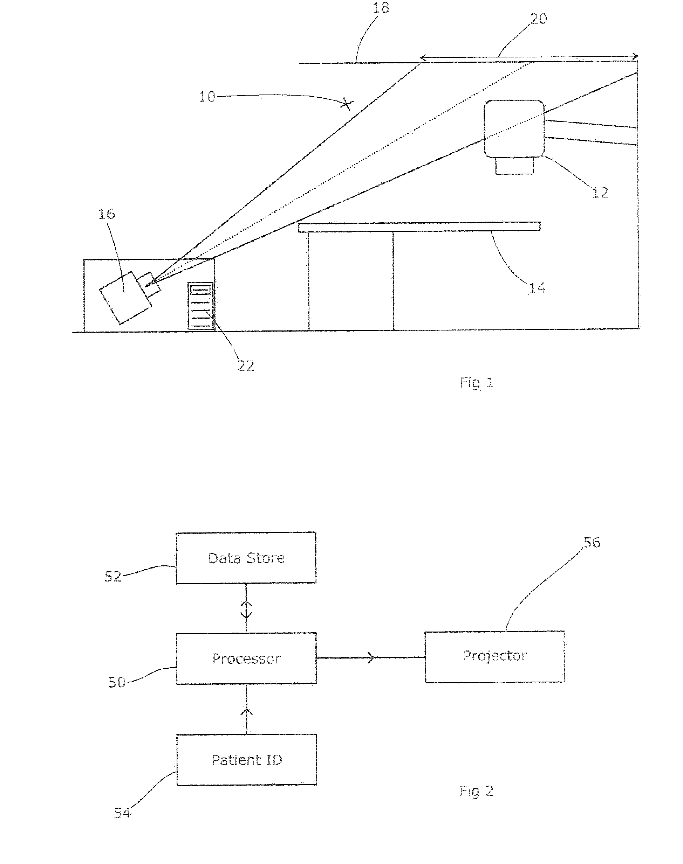

Figure 1 shows a suitable apparatus for creating the above-described ambience. A treatment room 10 contains a radiotherapy apparatus 12 and a patient support 14. A patient will typically recline on the support 14 while the radiotherapy apparatus 12 rotates around them and delivers the prescribed dose. Typical examples of a patient support and a radiotherapy apparatus are shown, but in practice each may be modified or replaced as required by the specific context.

A projector 16 is located within the treatment room 10 and is arranged so as to project an image onto a ceiling 18 of the room 10 in the region 20 above the patient table 14. This is the area of the ceiling 18 of which the patient will have a view when reclining on the patient support 14, meaning that an image projected by the projector 16 will be visible to the patient. A computer 22 controls the projector 16 in the manner described above and supplies it with the appropriate images etc.

The projector is shown within the treatment room 10, but may alternatively be located elsewhere provided it is able to project an image visible within the treatment room. For example, the projector 16 could be located in an adjacent room, projecting through a transparent window. Alternatively, the projector 16 could be above the ceiling or behind a false wall, projecting onto the rear of a translucent panel with the front being visible to the patient. Also, the projector is shown projecting an image directly onto the ceiling 18, but if desired mirrors, lenses and the like could be interposed in order to control the location of the image relative to that of the projector 16.

The projector 16 is shown projecting an image onto the ceiling obliquely, which will normally introduce distortions into the projected image. Either the computer 22 or the projector 16 (or the two acting together) should therefore introduce a counter-distortion in order to ensure that the desired image is displayed.

Figure 2 shows the general system layout. A processor unit 50 has read/write access to a data store 52 which holds suitable images, animation, audio and video files for creating the required ambiences, and read access to a patient ID system 54 which provides the processor with a unique identifier for the current patient. This may be a biometric scanning device which detects a unique biometric marker for the patient such as their fingerprint or their iris image, or an identity document scanning device able to scan a suitable identity document such as a State identity card, drivers licence or passport, or a human-controlled system such as a receptionist with a computer terminal who can identify the patient and input their identity to the system. The processor then uses the patient ID to select the correct ambience and/or the correct point within the ambience, and supplies the necessary audio and/or video files to the projector 56.

The processor unit 50 may also have access to the room environment controls (not shown) to enable it to set the room lighting including brightness levels and lighting pattern, room temperature, and room humidity according to a preference stored in association with the patient record.

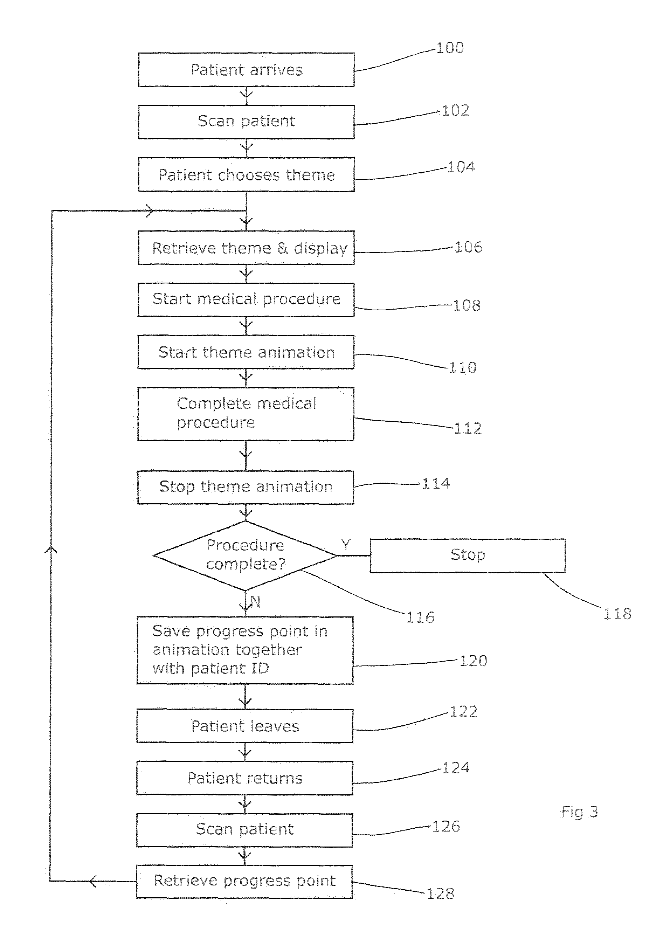

Figure 3 shows a flowchart for the process of a treatment. A patient arrives at the clinic (step 100) and is identified (step 102) by the patient ID system 54 to yield a unique patient ID. Assuming that this is the first visit by that patient, they choose a theme (step 104) from a range of available themes, and the processor 50 retrieves that theme from the data store 52 and displays the them (step 106) via the projector 56. The medical procedure is started (step 108) and any animated component of the theme is commenced (step 110).

In time, the medical procedure (such as a treatment fraction of a radiotherapy treatment) will be completed (step 112) and the processor will therefore halt any animated component of the theme (step 114). The processor then checks to see if the overall medical procedure is complete, for example whether all treatment fractions of a radiotherapy treatment have been delivered (step 116) and, if so, the process stops (step 118). If the overall procedure is not complete, then the processor unit 50 saves the theme choice and progress point together with the patient ID in the data store 52 (step 120). The patient then leaves (step 122).

At a later time or date, the patient returns for the next fraction or next stage of the medical procedure (step 124). The patient is scanned again (step 126) or otherwise identified, and the processor unit 50 uses the patient ID thus obtained to retrieve the theme choice and progress point. The process then repeats from the theme being displayed (step 106) and the medical procedure re-starting (step 108).

Often, the patient being treated will be within a bore of an MRI or similar apparatus with very little view of the rest of the room. This has a number of implications. As the patient will usually have a combination of ear defenders and headphones on, there can be a significant auditory contribution to the ambience, replayed at a volume level retained as a preference stored in association with the patient record. The system can then combine visual effects when the patient is entering the room and being set up, switching to audio effects when they are in the treatment position, and then back to visual effects when they come out. The two types of effect could of course linked, so the patient might for example go into the tube "on Earth", have an audio "space transit to the moon", and when they come out be on a lunar landscape, or some such. Then when they return for the next fraction, the room will be set to the moon setting, and they could experience a transit to Mars, and so on.

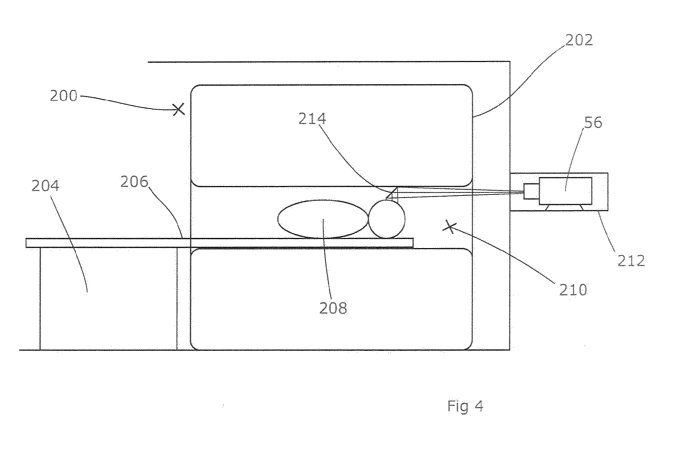

Alternatively, or in addition, a system for projecting the images inside the bore would allow the patient to continue seeing them throughout the treatment. Systems currently exist which employ mirrors mounted to non-magnetic spectacle-like frames, enabling the patient to view down the bore to a screen mounted outside. An implementation employing such a system is shown in figure 4 in which the treatment room 200 houses an MRI-based device 202 such as an MRI scanner, or combined MRI/Radiotherapy apparatus. A patient support system 204 has a sliding bed 206 onto which a patient 208 can recline; the bed 206 then slides into a bore 210 of the MRI-based device 202 as shown, placing the patient 208 headfirst within the bore 210.

The projector 56 is supported so that it is able to project an image down the bore 210. As shown in figure 4, the projector 56 projects an image from the head end of the bore 210, i.e. from the opposite end from which the patient was inserted. This means that the patient 208 can be inserted into or withdrawn from the bore 210 without interference with the projector 56. Alternatively, if access to that end of the bore 210 is problematic, the projection could be placed at the accessible end of the bore 210 but may have to be moved to allow the patient to be inserted or withdrawn. In this case, the projector 56 is supported in a recess 212 formed at the correct height, but could be supported otherwise such as by a shelf, or tripod, or suspension system or the like.

A mirror 214 is then provided for the patient, which may be mounted to an interior face of the bore 210 or to the patient via a spectacle-like set of frames. Thus enables the patient to see the output of the projector 56. Audio accompaniment may also be provided by headphones leading into the bore 210, or the like.

It may be problematic to predict exactly how long a specific fraction will be for each patient in enough time to procure a recording of a suitable length. However, given that there is a typical minimum time of approximately 5 minutes, and a typical maximum time of approximately 10 or 15 minutes, it is feasible to commission stories of approximately 5 minutes in length, or in groups of 5 and 10 minutes in length, and then provide an algorithm that inserts gaps of a few seconds between sections, either silent or with a filler sound such as music. In this way, a base recording could be stretched according to how long the specific treatment fraction is going to be.

In the case of the space travel example given above, appropriate points in the story could be flagged prior to processing, and a space-themed background track could be prepared to fill these gaps. The algorithm would then look at how much extra time needed to be filled so that the story ended at the same time as the treatment, divide that time equally between the gaps, and play the theme music in those gaps. This would make the story last the same time as the treatment, without being excessively irksome for the patient.

Thus, the above-described system achieves a pleasant and personalised ambience within the treatment room, reassuring and welcoming the patient when they return for the next treatment fraction, and providing a distraction for them during the fraction. It will of course be understood that many variations may be made to the above-described embodiment without departing from the scope of the present invention.

## Claims

1. A method of adjusting an environment in the context of a repeating medical procedure, comprising:
providing (i) a treatment room in which the medical procedure can take place,
the room comprising a display means, (ii) a data store, associated with the display means, and containing a plurality of displayable themes, and (iii) a patient identification means;
identifying the patient via the patient identification means;
checking whether records of a previous visit by that patient exist;
if no such records exist,
i. allowing the patient to select one theme from the plurality of themes
ii. admitting the patient into the treatment room, commencing display of the one theme via the display means, and commencing a medical procedure;
iii. ceasing the medical procedure and the display of the one theme, and recording an identifier of the patient together with a an identification of the one theme;
if a record of a previous visit exists,
i. retrieving the stored identification of the one theme
ii. re-admitting the patient into the treatment room, re-commencing display of the one theme, and re-commencing the medical procedure.

2. A method according to claim 1 in which the patient identification means consists of at least one of a biometric scanning device, an identity document scanning device, and a receptionist with a computer terminal.

3. System for personalisation of a medical environment, comprising:
a patient support,
a medical apparatus able to deliver a medical procedure to a patient located on the patient support,
a display means,
a data store, associated with the display means, containing a plurality of displayable themes,
a patient identification means, and
a control means programmed to identify the patient via the patient identification means, check whether records of a previous visit by that patient exist, and
i. if no such records exist, allow the patient to select one theme from the plurality of themes, admit the patient into the treatment room, commence display of the one theme via the display means, and commence a medical procedure, subsequently cease the medical procedure and the display of the one theme, and record in the data store an identifier of the patient together with a an identification of the one theme;
ii. if a record of a previous visit exists, retrieve the stored identification of the one theme, re-admit the patient into the treatment room, re-commence display of the one theme, and re-commence the medical procedure.

4. A treatment room according to claim 3 in which the medical apparatus is one selected from the group consisting of a CT scanner, an MRI scanner, a radiotherapy apparatus, and an apparatus combining the functions of at least two of the aforesaid.

5. A method of adjusting an environment in the context of a repeating medical procedure, comprising:
providing (i) a treatment room in which the medical procedure can take place, the room comprising a display means, and (ii) a data store, associated with the display means, and containing at least one displayable sequence;
admitting a patient into the treatment room, commencing display of the displayable sequence via the display means, and commencing a medical procedure;
ceasing the medical procedure and the display of the displayable sequence, and recording an identifier of the patient and a progress point within the displayable sequence;
subsequently, re-admitting the patient into the treatment room, re-commencing display of the displayable sequence from the recorded progress point, and re-commencing the medical procedure.

6. A method according to any one of claims 1, 2 or 5 in which the display means is a projector.

7. A method according to claim 6 in which the projector projects the theme or displayable sequence onto at least one of a wall and a ceiling.

8. A method according to any one of claims 1, 2 or 5 in which the display means is a wall-mounted screen.

9. A method according to any one of claims 1, 2 or 5 to 8 in which the theme or displayable sequence includes a progress indicator for the medical procedure.

10. A method according to claim 9 in which the progress indicator reflects the progress of the part of the medical procedure relevant to the current visit.

11. A method according to claim 9 or claim 10 in which the progress indicator reflects the progress of the medical procedure as a whole.

12. A method according to any one of claims 1, 2 or 5 to 11 in which an audio sequence is played via an audio output in conjunction with display of the theme or displayable sequence.

13. A method according to claim 12 in which the audio sequence has a length less than that of the medical procedure and is manipulated by inserting segments thereby to extend its length so as substantially to match the length of the medical procedure.

14. A method according to any one of claims 1, 2 or 5 to 13 in which the medical procedure is a radiotherapy treatment.

15. A method according to any one of claims 1, 2 or 5 to 13 in which the medical procedure is one of a CT and an MRI scan.

16. A method according to any one of claims 1, 2 or 5 to 13 in which the medical procedure is a combination of one of a CT and an MRI scan with a radiotherapy treatment.

17. System for personalisation of a medical environment, comprising:
a patient support,
a medical apparatus able to deliver a medical procedure to a patient located on the patient support,
a display means,
a data store, associated with the display means, containing a displayable sequence,
a patient identification means, and
a control means programmed to commence display of the displayable sequence via the display means, and commence a medical procedure, subsequently cease the medical procedure and the display of the displayable sequence, and record in the data store an identifier of the patient and a progress point within the displayable sequence, and subsequently re-commence display of the displayable sequence from the recorded progress point, and re-commence the medical procedure.
